# FASCICULE DE BREVET EUROPEEN

(11) **EP 1 919 392 B1**
(45) Date de publication et mention de la délivrance du brevet: **16.05.2012**
(21) Numéro de dépôt: 06794527.9
(22) Date de dépôt: 03.08.2006
(51) Int. Cl.: A61F 2/00

(54) **PROTHESE DE RENFORT SOUS FORME D'UNE STRUCTURE TEXTILE**
VERSTÄRKUNGSPROTHESE EINER TEXTILEN STRUKTUR
REINFORCING PROSTHESIS OF TEXTILE STRUCTURE

(30) Priorité: 04.08.2005 FR 0552430
(43) Date de publication de la demande: 14.05.2008
(73) Titulaire: Microval, 43240 Saint Just Malmont (FR)
(72) Inventeur: CUILLERON, Olivier, F-42270 Saint Priest en Jarez (FR); PAIN, Bernard, F-43120 Monistrol sur Loire (FR)
(74) Mandataire: Thivillier, Patrick
(86) Numéro de dépôt international: PCT/FR2006/050786
(87) Numéro de publication internationale: WO 2007/015033

(56) Documents cités:
- WO-A-02/07648
- WO-A-2005/044143
- US-A- 5 316 543
- US-A1- 2003 212 460
- US-A1- 2004 181 288
- US-B1- 6 669 735

## Description

Plus particulièrement, l'invention concerne une prothèse pour hernie hiatale.

On rappelle d'une manière parfaitement connue pour un homme du métier et pour une meilleure compréhension de la suite de la description qu'une hernie hiatale est le passage permanent ou intermittent d'une portion de l'estomac à travers l'orifice oesophagien du diaphragme.

Ce passage de l'extrémité supérieure de l'estomac dans l'orifice hiatal survient si les moyens de liaison entre l'estomac et le bas de l'oesophage sont défaillants. Dans plus de 90 % des cas, il y a un glissement vers le haut de la partie haute de l'estomac (cardia) qui a tendance à quitter l'abdomen pour se retrouver dans le thorax. Les causes en sont par exemple l'augmentation de la pression dans l'abdomen, une obésité, ...

Dans les cas restants, il y a généralement enroulement de la grosse tubérosité de l'estomac qui passe à travers l'orifice diaphragmatique alors que le bas de l'oesophage et le haut de l'estomac restent en place. La cause en est le plus souvent un défaut congénital de l'orifice oesophagien du diaphragme, éventuellement un traumatisme.

Pour traiter ce type de hernie, le traitement chirurgical n'est indiqué que s'il existe un reflux important et gênant malgré un traitement bien conduit ou en cas d'oesophagite grave. Pour l'essentiel, le traitement chirurgical consiste à replacer correctement l'estomac sous le diaphragme, puis à resserrer l'orifice hiatal, et enfin à reconstituer un dispositif anatomique afin de s'opposer au reflux. Ce dispositif anatomique peut être du type Nissen (fundoplicature complète) ou du type Toupet (hémi-fundoplicature). Sans ouvrir le tube digestif, le chirurgien fabrique une valve formée par la paroi de la grosse tubérosité pour manchonner l'oesophage abdominal.

A partir de cet état de la technique, on a proposé d'apporter une alternative chirurgicale utilisant une prothèse apte à assurer un remodelage de l'orifice oesophagien du diaphragme. Une solution ressort par exemple de l'enseignement du document US 2004/0181288 qui concerne une prothèse pour hernie hiatale sous forme d'une structure textile présentant un orifice à placer autour de l'oesophage. La structure de la prothèse est réalisée à partir de deux couches superposées et assemblées, de sorte que l'on ne peut exclure des risques de désolidarisation. Il en résulte également un manque de souplesse. On observe par ailleurs que le pourtour de l'orifice en contact avec l'oesophage peut créer un effet traumatique. Le document US-A-2003/0212460 décrit une prothèse selon le préambule de la revendication 1.

L'invention s'est fixée pour but de remédier à ces inconvénients, d'une manière simple, sure, efficace et rationnelle.

Le problème que se propose de résoudre l'invention à partir d'une prothèse du type de celle définie par l'enseignement du document US 2004/0181288 est d'éviter tout effet traumatique, d'assurer un remodelage automatique sans tension et d'obtenir un serrage optimal de l'oesophage, afin d'éviter toute dysphagie ou récidive.

Pour atteindre ces objectifs, selon l'invention, il a été conçu et mis au point une prothèse de renfort pour hernie hiatale sous forme d'une structure textile présentant un orifice à placer autour de l'oesophage, une fente étant formée depuis ledit orifice jusqu'au bord externe de la structure de manière à délimiter deux branches. La structure est réalisée dans un matériau souple et biocompatible 100 % synthétique apte à s'adapter à la forme anatomique de la zone à traiter, l'une des faces de ladite structure étant imprégnée d'un film en matière non adhérente. Un bourrelet de section arrondie et réalisé dans une matière non adhérente par rapport à l'oesophage, est surmoulé au niveau du bord annulaire de l'orifice, afin de ne pas créer de traumatisme vis-à-vis de l'oesophage. Les extrémités libres des branches présentent des trous pour permettre un serrage en position de chevauchement réglable desdites branches pour permettre de régler le diamètre de l'orifice en fonction de l'oesophage. La structure présente des microporosités afin d'évacuer les séromes et hématomes.

Avantageusement, la structure est réalisée en polypropylène ou polyester tricoté ou non tissé.

Selon une autre caractéristique, la structure présente des trous indiquant le passage des sutures.

Pour résoudre le problème posé de parfaitement respecter l'anatomie, la fente de l'orifice est décalée angulairement par rapport à l'axe de symétrie de la structure.

Avantageusement, le film et le bourrelet, en matière non adhérente, sont composés de silicone ou polyuréthane.

L'invention est exposée ci-après plus en détail à l'aide des figures des dessins annexés dans lesquels :
- la figure 1 est une vue de face de la structure de la prothèse selon l'invention ;
- la figure 2 est une vue en coupe considérée selon la ligne 2-2 de la figure 1 ; pour une meilleure compréhension des dessins, les dimensions et épaisseurs ont volontairement été exagérées ;
- les figures 3 et 4 montrent la technique opératoire pour la mise en place de la prothèse selon l'invention.

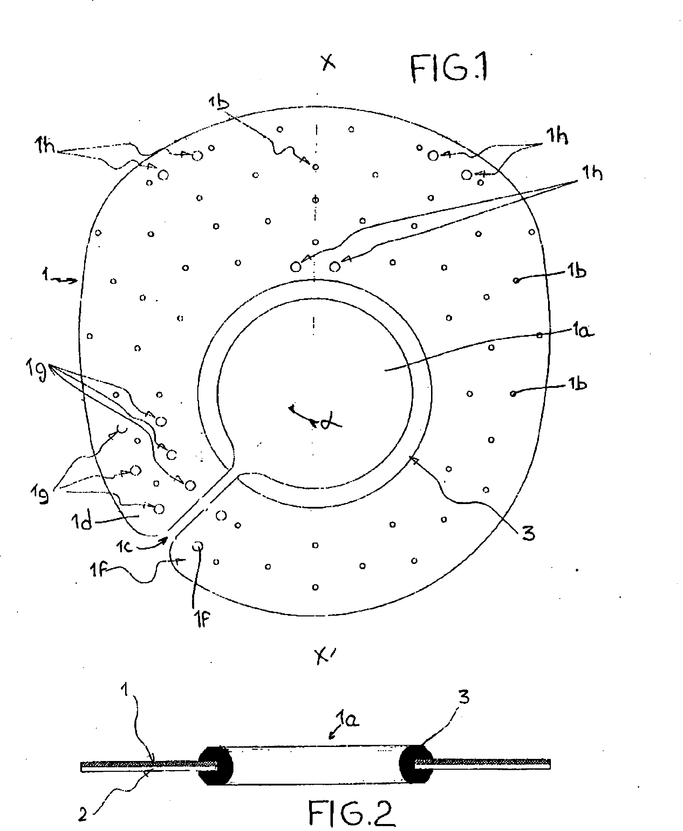

Selon l'invention, la prothèse pour hernies hiatales, est réalisée à partir d'une structure textile (1) présentant un orifice (1a) destiné à être disposé autour de l'oesophage (O). La structure (1) est réalisée dans un matériau biocompatible composite bifaces 100 % synthétique, notamment en polypropylène ou polyester tricoté ou non tissé. L'ensemble de la structure (1) est souple et non élastique et a une forme générale déterminée pour s'adapter à l'anatomie de la zone à traiter. Cette forme anatomique ressort de la figure 1 des dessins annexés.

Les faces de la structure sont recouvertes, notamment par imprégnation d'un film (2) en matière non adhérente notamment vis-à-vis des viscères. Par exemple, ce film (2) est composé de silicone ou de polyuréthane.

La structure (1) présente des microporosités (1b) afin d'évacuer les séromes et hématomes.

Afin de permettre la mise en place de l'ensemble de la structure (1) par rapport à l'oesophage, une fente (1c) est formée depuis l'orifice (1a) jusqu'au bord externe de la structure, de manière à délimiter deux branches (1d) et (1e) susceptibles d'être écartées. De même, ces dispositions permettent de régler le diamètre de l'orifice (1a) en fonction de l'oesophage, par chevauchement des extrémités libres des branches (1d) et (1e). Dans ce but, des trous (1f) et (1g) formés à l'extrémité de chacune des branches (1d) et (1e), permettent un serrage optimal de l'oesophage afin d'éviter toute dysphagie ou récidive. En fonction des trous choisis, le diamètre de l'orifice est calibré de manière précise.

Suivant une caractéristique importante de l'invention, l'orifice (1a) est délimité, au niveau de sa zone en contact avec l'oesophage par un bourrelet (3) conformé en section afin de ne pas créer des traumatismes vis-à-vis de l'oesophage. Ce bourrelet (3) est de section arrondie et est surmoulé au niveau du bord annulaire de l'orifice (1a). Le bourrelet (3) est réalisé dans une matière non adhérente par rapport à l'oesophage, par exemple en étant composé de silicone ou de polyuréthane. De par sa conformation, compte tenu de la nature du matériau le composant, le bourrelet (3) en contact avec l'oesophage, évite tout risque traumatique vis-à-vis de ce dernier.

Toujours en ayant pour objectif de respecter l'anatomie et de faciliter la mise en place de la prothèse, la fente (1c) est orientée angulairement selon un angle (α) par rapport à l'axe de symétrie (X-X') de la structure.

Des trous (1h) sont convenablement disposés par rapport à l'axe de symétrie (X-X') de la structure, afin d'indiquer le passage des sutures.

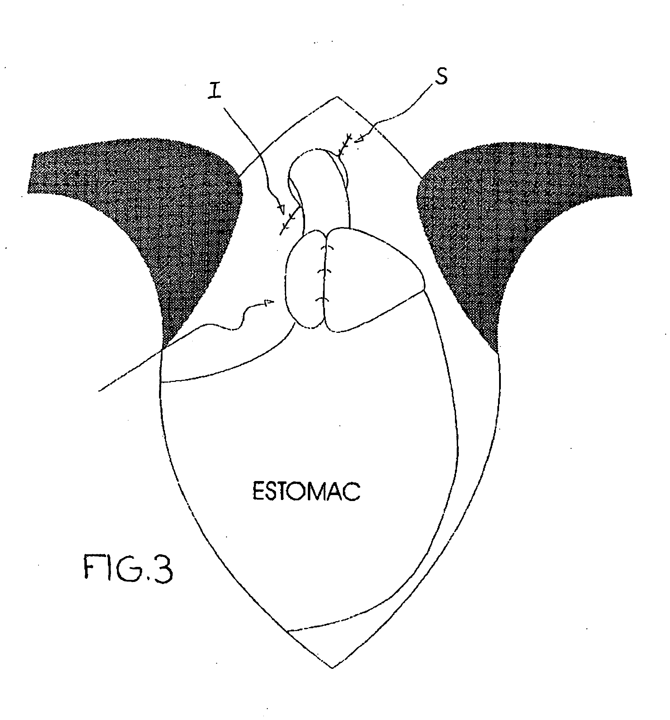

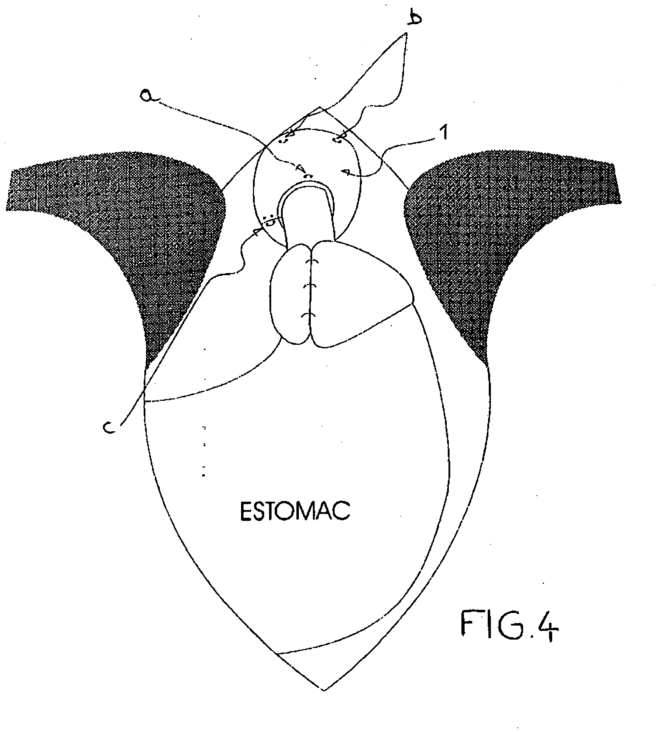

Pour la mise en place de la prothèse, la technique opératoire est la suivante :
- On réalise la dissection de l'orifice oesophagien ainsi que sa fermeture. La fermeture est effectuée avec seulement deux points en partie inférieure (I), le reste des points étant réalisés à la partie supérieure (S) au-dessus de l'oesophage. On réalise une valve anti-reflux par fundoplicature totale ou partielle selon la technique habituelle choisie (figure 3).
- On introduit ensuite la prothèse (1) dans la cavité abdominale en présentant la face de la structure non revêtue du film (2) en contact avec le diaphragme et la face revêtue du film (2) en contact avec les viscères abdominaux.
- On maintient le positionnement de la structure par un premier point de suture (a) sur la partie supérieure la plus proche de l'orifice oesophagien.
- On réalise ensuite les points de suture latéraux supérieurs (b) disposés au niveau de la partie la plus large de la prothèse, de façon à couvrir la partie du diaphragme où se produisent le plus fréquemment les récidives.
- On entoure l'oesophage par l'arrière avec la branche gauche (1d) que l'on croise jusqu'à la branche (1e) afin de réaliser les points d'union des deux branches en choisissant une des différentes positions

possibles en mettant en correspondance les trous (1g) et (1f) de manière à laisser l'orifice hiatal à la taille appropriée.
- On commence le point dans l'orifice situé du côté interne de la position choisie de la branche gauche (1d) et on place ensuite une aiguille dans l'orifice situé du côté interne de l'autre branche (1e).
- L'aiguille traverse ensuite les deux piliers musculaires de l'orifice oesophagien du diaphragme entre les points d'approche précédemment effectués. L'aiguille sort d'abord par l'orifice externe de la branche gauche (1e) et passe par l'orifice externe de la branche (1d), se nouant ainsi sans s'étrangler en insérant les muscles du pilier.
- Enfin, on place le dernier point central (c) de suture au-dessus des orifices déjà marqués dans la prothèse afin de terminer son positionnement (figure 4).

Les avantages ressortent bien de la description, en particulier on souligne et on rappelle :
- l'utilisation de cette prothèse permet un remodelage anatomique sans tension de l'orifice oesophagien du diaphragme ;
- l'utilisation d'une structure biface bio-compatible 100 % synthétique avec une face en polypropylène ou polyester tricoté ou non tissé et une autre face imprégnée d'un film en matière non adhérente ;
- la réalisation d'un bourrelet non traumatique au niveau du bord annulaire de l'orifice en contact avec l'oesophage ;
- la possibilité de régler le diamètre de l'orifice pour obtenir un serrage optimal de l'oesophage afin d'éviter toutes dysphagies ou récidives.

## Revendications

1. Prothèse de renfort pour hernie hiatale sous forme d'une structure textile (1) présentant un orifice (1a) à placer autour de l'oesophage, une fente (1c) étant formée depuis ledit orifice (1a) jusqu'au bord externe de la structure de manière à délimiter deux branches (1d) et (1e),
la structure (1) est réalisée dans un matériau souple et biocompatible 100% synthétique apte à s'adapter à la forme anatomique de la zone à traiter et présente des microporosités (1b) afin d'évacuer les séromes et hématomes,
**caractérisé en ce que** :
- un bourrelet (3), de section arrondie est surmoulé au niveau du bord annulaire de l'orifice (1a), afin de ne pas créer de traumatismes vis-à-vis de l'oesophage;
- le bourrelet est réalisé dans une matière non adhérente par rapport à l'oesophage et l'une des faces de la structure est imprégnée d'un film (2) en matière non adhérente.
- les extrémités libres des branches (1d) et (1e) présentent des trous (1f) et (1g) pour permettre un serrage en position de chevauchement réglable desdites branches pour permettre de régler le diamètre de l'orifice (1a) en fonction de l'oesophage;
- la fente (1c) de l'orifice (1a) est décalée angulairement par rapport à l'axe de symétrie de la structure (1).

2. Prothèse selon la revendication 1, **caractérisée en ce que** la structure (1) est réalisée en polypropylène ou polyester tricoté ou non tissé.

3. Prothèse selon la revendication 1, **caractérisée en ce que** la structure (1) présente des trous (1h) indiquant le passage des sutures.

4. Prothèse selon la revendication 1, **caractérisée en ce que** le film (2) et le bourrelet (3) en matière non adhérente, sont composés de silicone ou polyuréthane.

## Claims

1. Reinforcement prosthesis for a hiatal hernia in the form of a textile structure (1) having an orifice (1a) to be placed round the oesophagus, a slit (1c) running from the said orifice (1a) to the outer edge of the structure so as to delimit two branches (1d) and (1e),
the structure (1) being made of a supple and 100% biocompatible synthetic material capable of adapting to the anatomical shape of the area to be treated and having microporosities (1b) in order to evacuate seromas and hematomas,
**characterised in that**:
- a bead (3), having a rounded section, is overmoulded onto the annular edge of the orifice (1a), so as to prevent trauma to the oesophagus;
- the bead is made of a material which is non-adhesive in relation to the oesophagus and one of the faces of the structure is impregnated with a film (2) of non-adhesive material;
- the free ends of the branches (1d) and (1e) have holes (1f) and (1g) to allow the said branches to be secured in an adjustable overlapping position so as to allow the diameter of the orifice (1a) to be adjusted to suit the oesophagus;
- the slit (1c) of the orifice (1a) is offset at an angle in relation to the axis of symmetry of the structure (1).

2. Prosthesis according to claim 1, **characterised in that** the structure (1) is made of knitted or non-woven polypropylene or polyester.

3. Prosthesis according to claim 1, **characterised in that** the structure (1) has holes (1h) indicating the passage of sutures.

4. Prosthesis according to claim 1, **characterised in that** the film (2) and the bead (3) of non-adhesive material are composed of silicone or polyurethane.

## Patentansprüche

1. Verstärkungsprothese für Hiatushemie in Form einer Textilstruktur (1) mit einer um die Speiseröhre herum zu platzierenden Öffnung (1a) und einem Schlitz (1c), der von der besagten Öffnung (1a) aus bis zum Außenrand der Struktur ausgebildet ist, um zwei Schenkel (1d und 1e) abzugrenzen,
wobei die Struktur (1) aus einem weichelastischen und 100% biokompatiblen synthetischen Material gefertigt ist, das geeignet ist, sich an die anatomische Form der zu behandelnden Zone anzupassen, und Mikroporositäten (1b) aufweist, um Serome und Hämatome zu evakuieren,
**dadurch gekennzeichnet, dass**
- ein Wulst (3) mit abgerundetem Querschnitt im Bereich des ringförmigen Randes der Öffnung (1a) aufgespritzt ist, um keine Traumata gegenüber der Speiseröhre hervorzurufen;
- der Wulst aus einem an der Speiseröhre nicht haftenden Material besteht und eine der Seitenflächen der Struktur mit einer Folie (2) aus einem nichthaftenden Material imprägniert ist;
- die freien Enden der Schenkel (1d und 1e) Löcher (1f und 1g) aufweisen, um ein Festziehen in der regulierbar überlappenden Position der besagten Schenkel zu ermöglichen und so den Durchmesser der Öffnung (1a) in Abhängigkeit von der Speiseröhre zu regulieren;
- der Schlitz (1c) der Öffnung (1a) gegenüber der Symmetrieachse der Struktur (1) winkelig versetzt ist.

2. Prothese nach Anspruch 1, **dadurch gekennzeichnet, dass** die Struktur (1) aus Polypropylen oder Polyester, gestrickt oder als Vliesstoff, besteht.

3. Prothese nach Anspruch 1, **dadurch gekennzeichnet, dass** die Struktur (1) Löcher (1h) als Anzeige für die Durchführung der Nähte aufweist.

4. Prothese nach Anspruch 1, **dadurch gekennzeichnet, dass** die Folie (2) und der Wulst (3) aus nichthaftendem Material aus Silikon oder Polyurethan bestehen.
